# EUROPÄISCHE PATENTSCHRIFT

(11) **EP 0 620 207 B1**
(45) Veröffentlichungstag und Bekanntmachung des Hinweises auf die Patenterteilung: **13.08.1997**
(21) Anmeldenummer: 94105455.3
(22) Anmeldetag: 08.04.1994
(51) Int. Cl.: C07C 67/52, C07C 67/54, C07C 69/76

(54) **Gewinnung von 4,4'-Biphenyl-dicarbonsäure-dimethylester**
Recovery of 4,4'-biphenyldicarboxylic and dimethyle ester
Production de l'ester diméthylique de l'acide biphényldicarboxylique

(30) Priorität: 16.04.1993 DE 4312491
(43) Veröffentlichungstag der Anmeldung: 19.10.1994
(73) Patentinhaber: HOECHST AKTIENGESELLSCHAFT, 65929 Frankfurt am Main (DE)
(72) Erfinder: Frey, Michael Dr., D-86356 Neusäss (DE); Hertenstein, Ulrich Dr., D-86456 Gablingen (DE); Schaller, Rainer Dr., D-86637 Wertingen (DE)

(56) Entgegenhaltungen:
- DE-A- 2 310 824
- US-A- 3 277 153

## Beschreibung

Die vorliegende Erfindung betrifft die Gewinnung von 4,4'-Biphenyl-dicarbonsäure-dimethylester aus Rückständen der Herstellung von Dimethyl-terephthalat.

Dimethylterephthalat (= DMT) ist ein großtechnisch hergestelltes, wertvolles Ausgangsmaterial, das in großem Umfang zur Herstellung von Polyesterfasern eingesetzt wird. Es wird durch katalytisch Oxydation von Xylol, Umsetzung des rohen Oxydationsgemisches mit Methanol und anschließende fraktionierte Destillation des so hergestellten, rohen Dimethylterephthalats gewonnen.
Bei dieser Destillation bleibt in der Destillierblase ein dunkler, zäher Rückstand zurück, der entsorgt werden muß, was bisher vorzugsweise durch Verbrennung erfolgte.

Dem Fachmann ist es bekannt, daß dieser Rückstand eine komplexe Mischung verschiedener Nebenprodukte der DMT-Produktion darstellt, die typischerweise 0,5 bis 10 Gew.-% 4,4'-Biphenyl-dicarbonsäure-dimethylester enthält. 4,4'-Biphenyl-dicarbonsäure-dimethylester ist auch in anderen Produkt-Seitenströmen von DMT-Anlagen (z.B. Destillat der Methanolyse) in ähnlichen Anteilen vorhanden.

In der US-A-3 277 153 wird ein Verfahren beschrieben zur Gewinnung von Diphenyl-polycarbonsäure-methylestern neben 3,4-Benzocumarin-dicarbonsäure-dimethylester aus den bei der Dimethylterephthalat-Herstellung anfallenden Destillationsrückständen. Bei diesem Verfahren wird ein Destillationsrückstand, aus dem alle Anteile von Terephthalsäure- bzw. Isophthalsäure-dimethylester durch Destillation bei vermindertem Druck abgetrennt wurden, in Methanol oder Xylol in der Hitze digeriert. Unlösliche Anteile werden abgetrennt, die erhaltene klare Lösung vom Lösungsmittel befreit und die erhaltenen Extrakte in an sich bekannter Weise zu chemisch einheitlichen Stoffen aufarbeitet.
Dieses Verfahren ist äußerst aufwendig, da, wie in dieser Druckschrift beschrieben, mehrfaches Umkristallisieren aus verschiedenen Lösungsmitteln zur Isolierung von reinem 4,4'-Biphenyl-dicarbonsäure-dimethylester erforderlich ist. Weiterhin finden sich in der Regel in den so erhaltenen Produkten unerwünschte Reste von Oxydationskatalysatoren.

In der DE-A-23 10 824 wird die Isolierung von 4,4'-Biphenyl-dicarbonsäure-dimethylester aus Rückständen der DMT-Produktion durch Destillation und Kristallisation nach einer obligatorischen Wärmebehandlung beschrieben. Es wird dabei hervorgehoben, daß ohne diese Wärmebehandlung eine große Menge von unerwünschten Verbindungen entsteht sodaß die Isolierung reinen 4,4'-Biphenyl-dicarbonsäure-dimethylesters schwierig ist.
Ein sehr gravierender Nachteil dieses Verfahrens besteht darin, daß der danach erhaltene 4,4'-Biphenyl-dicarbonsäure-dimethylester mit ca. 9 Gew.-% Trimethyl-2,4',5-biphenyl-tricarboxylat verunreinigt ist. Diese Verunreinigung ist für die Herstellung von Polyestern äußerst schädlich, da sie bereits in Spuren zu Verzweigungen und Vernetzungen führt und damit das Polymer unbrauchbar macht. Diese Verunreinigung muß durch wiederholtes Auswaschen des Produkts mit einem Lösemittel entfernt werden, wobei stets ein sehr unvorteilhafter Kompromiss zwischen Substanzverlust und Reinheit geschlossen werden muß.

4,4'-Biphenyl-dicarbonsäure-dimethylester ist ein wertvolles Ausgangsmaterial für Synthesen und kann beispielsweise auch bei der Herstellung von Polyestern als Modifizierungsmittel zur gezielten Beeinflussung des Eigenschaftsprofils dieser Polymere und somit als Ausgangsmaterial für Polyester mit verbesserten Eigenschaften der daraus hergestellten Formkörper, insbesondere von daraus hergestellten Fasern, Folien, oder durch thermoplastische Verarbeitung hergestellten Formteilen dienen.

Für die Herstellung von Polyestern ist es entscheidend, Rohstoffe von hoher Reinheit einzusetzen, da andernfalls keine ausreichend hohen Molekulargewichte erhalten werden können und unerwünschte Verzweigungen und Vernetzungen auftreten.

4,4'-Biphenyl-dicarbonsäure-dimethylester und seine Herstellung ist an sich bekannt. Eine Übersicht über die Bildung und Herstellung von 4,4'-Biphenyl-dicarbonsäure-dimethylester findet sich in "Beilsteins Handbuch der organischen Chemie", Band 9(I), Seite 927; Band 9(II), Seite 665 und Band 9(III), Seite 4519. Danach wird 4,4'-Biphenyl-dicarbonsäure-dimethylester am günstigsten durch eine mehrstufige Synthese hergestellt, wobei 4,4'-Dimethyl-biphenyl ("Ditolyl") zunächst mit Bichromat/Schwefelsäure zu 4-Methyl-biphenyl-4'-carbonsäure und diese mit alkalischer Permanganatlösung zur 4,4'-Biphenyl-dicarbonsäure oxydiert wird und letztere mit Methanol verestert wird.

Es wurde nun überraschenderweise gefunden, daß aus dem bei der Herstellung von Dimethylterephthalat anfallenden Destillationsrückstand und aus Seitenströmen der DMT-Produktion 4,4'-Biphenyl-dicarbonsäure-dimethylester gewonnen werden kann.

Gegenstand der vorliegenden Erfindung ist somit ein Verfahren zur Gewinnung von 4,4'-Biphenyl-dicarbonsäure-dimethylester aus Rückständen und Seitenströmen der DMT-Herstellung, bei dem die Rückstände oder das Material der Seitenströme zunächst unter einem Druck von höchstens 7 mbar, vorzugsweise von höchstens 4 mbar, insbesondere bei 0,2 bis 3 mbar, bei einer Blasentemperatur von 200 bis 350 °C, vorzugsweise 200 bis 310 °C destilliert werden, wobei ein wachsartiges Destillat gewonnen wird, das bei einem Druck von beispielsweise 0,2 mbar im Bereich von 230 bis 270 °C übergeht und das weniger als 20 Gew.-%, vorzugsweise weniger als 10 gew.-% Rest-DMT enthält.

Dieses Destillat wird anschließend 10 bis 60 Minuten mit der 0,7- bis 10-fachen, vorzugsweise der 1- bis 5-fachen Gewichtsmenge eines organischen Lösungsmittels, gegebenenfalls unter Druck, (d.h. im geschlossenen Gefäß unter dem bei der Behandlungstemperatur sich einstellenden Dampfdruck des Lösungsmittels) bei einer Temperatur von 70 bis 180 °C, vorzugsweise von 70 bis 150 °C, insbesondere bei der Siedetemperatur des Lösungsmittels (unter Normaldruck) behandelt.
Die so erhaltene Lösung wird erforderlichenfalls von ungelösten, meist gallertartigen, Bestandteilen getrennt, z.B. durch Dekantieren, Filtrieren oder Zentrifugieren.
Die abgetrennte klare Lösung wird auf eine Temperatur von 45 bis 65 °C, vorzugsweise von 50 bis 55 °C abgekühlt und bei dieser Temperatur 0,5 bis 15 Std., vorzugsweise 0,5 bis 8 Std., kristallisieren gelassen, wobei eine Suspension von 4,4'-Biphenyl-dicarbonsäure-dimethylester - Kristallen erhalten wird, aus der die Kristalle auf üblichem Weg isoliert werden.
Zweckmäßigerweise werden die Kristalle durch Filtration, vorzugsweise auf Druck- oder Vakuumnutschen, oder durch Zentrifugieren aus der Suspension abgetrennt. Gegebenenfalls werden sie zur vollständigen Befreiung der Kristalle von adsorbierten Verunreinigungen ein- oder mehrfach mit dem gleichen oder einem anderen, vorzugsweise möglichst niedrig siedenden, organischen Lösungsmittel gewaschen. Anschließend werden die Kristalle getrocknet.

Als organische Lösungsmittel eignen sich für die Durchführung des erfindungsgemäßen Verfahrens niedere Alkanole mit 1 bis 8, vorzugsweise 1-4 C-Atomen, insbesondere Methanol, aber auch Erdölfraktionen mit einem Siedepunkt von ca. 50-150 °C, wie z.B. Petroläther oder Ligroin, Chlorkohlenwasserstoffe wie Methylenchlorid, Chloroform, Trichlorethylen, Perchlorethan oder auch Monochlorbenzol, lineare oder cyclische Ether wie z.B. Diethylether, Tetrahydrofuran oder Dioxan, niedere Ketone, wie z.B. Aceton oder Methy-ethylketon oder monocyclische aliphatische oder aromatische Lösemittel wie z.B. Cyclohexan, Benzol, Toluol oder Xylole.
Bevorzugt werden zur Durchführung des erfindungsgemäßen Verfahrens niedere Alkanole, insbesondere Methanol und Ethanol eingesetzt.
Der auf diese Weise erhaltene 4,4'-Biphenyl-dicarbonsäure-dimethylester hat nach gaschromatographischer Analyse eine Reinheit von über 99,8% und ist ohne weitere Reinigung zur Herstellung von Polyestern geeignet. Insbesondere ist der so hergestellte 4,4'-Biphenyl-dicarbonsäure-dimethylester frei von Rückständen der Oxydationskatalysatoren.
Wenn gewünscht kann dieser 4,4'-Biphenyl-dicarbonsäure-dimethylester jedoch durch Umkristallisieren aus geeigneten Lösungsmitteln, wie z.B. Methanol oder Xylol noch weiter, z.B. auf über 99,9% gereinigt werden.

Die folgenden Ausführungsbeispiele veranschaulichen die Durchführung des erfindungsgemäßen Verfahrens.

### Beispiel 1

100 g des bei der Herstellung von Terephthalsäure-dimethylester anfallenden Rückstands werden bei 0,2·10⁻³ bar bis zu einer Badtemperatur von 300 °C destilliert. Man erhält bei einer Übergangstemperatur von 230 bis 270 °C ein gelbes wachsartiges Destillat in einer Menge von 20,1 g.

Dieses Destillat wird mit 75 ml Methanol 30 Min. unter Rückfluß zum Sieden erhitzt. Die erhaltene Methanollösung wird von einem gallertartigen Bodenkörper abdekantiert und auf 50°C abgekühlt. Man läßt die Lösung 1 Std bei dieser Temperatur kristallisieren, saugt die ausgeschiedenen Kristalle ab, wäscht sie mit wenig eiskaltem Methanol und Diethylether nach und trocknet sie im Vakuum.

Ausbeute: 2,5 g farblose Kristalle von 4,4'-Biphenyl-dicarbonsäure-dimethylester mit einem Schmelzpunkt von 212 bis 214 °C.

### Beispiel 2

830 kg Destillationsrückstand der DMT-Herstellung, der 1,9 Gew.-% 4,4'-Biphenyl-dicarbonsäure-dimethylester und noch 3 Gew.-% DMT enthält, wird bei einer von 200°C auf 290°C steigenden Badtemperatur im Vakuum von 1 bis 3 mbar destilliert, wobei 300 kg eines leuchtend gelben Destillats erhalten werden.
Das Destillat, das einen Erstarrungspunkt von ca. 110°C besitzt, wird mit 300 kg Methanol im geschlossenen Gefäß unter Druck auf 90°C erhitzt, wobei eine klare Lösung erhalten wird. Die Lösung wird auf 55 °C abgekühlt und 30 Minuten bei dieser Temperatur gerührt. Die dabei auskristallisierten Kristalle werden abfiltriert, zur Befreiung von anhaftenden Verunreinigungen 2 mal mit je 20 l Methanol gewaschen, und im Vakuum bei 50°C getrocknet.

Ausbeute: 3,9 kg farblose Kristalle von 4,4'-Biphenyl-dicarbonsäure-dimethylester vom Schmelzpunkt 215 °C. Die Reinheit (gaschromatographisch) beträgt 99,8 %.

### Beispiel 3

276 kg eines Destillats, das 2,8 Gew.-% 4,4'-Biphenyl-dicarbonsäure-dimethylester und 7,3 Gew.-% DMT enthält, werden mit 310 kg Methanol im geschlossenen Gefäß unter Druck auf 100°C erhitzt, wobei eine klare Lösung erhalten wird. Die Lösung wird auf 57 °C abgekühlt und 60 Minuten bei dieser Temperatur gerührt. Die dabei auskristallisierten Kristalle werden abfiltriert, zur Befreiung von anhaftenden Verunreinigungen 2 mal mit je 20 kg Methanol gewaschen, und im Vakuum bei 50°C getrocknet.
Ausbeute: 8,2 kg farblose Kristalle von 4,4'-Biphenyl-dicarbonsäure-dimethylester vom Schmelzpunkt 215 °C. Die Reinheit (gaschromatographisch) beträgt 99,85 %.

## Patentansprüche

1. Verfahren zur Gewinnung von 4,4'-Biphenyl-dicarbonsäure-dimethylester aus Rückständen und Seitenströmen der DMT-Herstellung, bei dem die Rückstände bzw. das Material der Seitenströme zunächst unter einem Druck von höchstens 7 mbar bei einer Blasentemperatur von 200 bis 350 °C destilliert werden, das Destillat (Siedebereich bei einem Druck von beispielsweise 0,2 mbar 230 bis 270 °C) anschließend 10 bis 60 Minuten mit der 0,7- bis 10-fachen Gewichtsmenge eines organischen Lösungsmittels bei einer Temperatur von 70 bis 180 °C behandelt, die klare Lösung erforderlichenfalls von ungelösten Bestandteilen getrennt, die abgetrennte klare Lösung auf eine Temperatur von 45 bis 65 °C abgekühlt und bei dieser Temperatur kristallisieren gelassen wird, wobei eine Suspension von 4,4'-Biphenyl-dicarbonsäure-dimethylester - Kristallen erhalten wird, aus der die Kristalle auf üblichem Weg isoliert, ein oder gegebenenfalls ein oder mehrfach mit dem gleichen oder einem anderen Lösungsmittel gewaschen, und getrocknet werden.

2. Verfahren nach Anspruch 1, dadurch gekennzeichnet, daß als organisches Lösungsmittel Alkanole mit 1 bis 8 C-Atomen eingesetzt werden.

3. Verfahren nach mindestens einem der Ansprüche 1 und 2, dadurch gekennzeichnet, daß als organisches Lösungsmittel Methanol eingesetzt wird.

4. Verfahren nach mindestens einem der Ansprüche 1 bis 3, dadurch gekennzeichnet, daß bei einer Blasentemperatur von 200 bis 310 °C destilliert wird.

5. Verfahren nach mindestens einem der Ansprüche 1 bis 4, dadurch gekennzeichnet, daß das Destillat mit der 1- bis 5-fachen Menge des organischen Lösungsmittels behandelt wird.

6. Verfahren nach mindestens einem der Ansprüche 1 bis 5, dadurch gekennzeichnet, daß die Behandlung des Destillats mit dem organischen Lösungsmittel bei 70 bis 150 °C erfolgt.

## Claims

1. A process for isolating dimethyl 4,4'-biphenyldicarboxylate from residues and secondary streams of DMT production, which comprises first distilling the residues or the material of the secondary streams at a pressure of at most 7 mbar at a boiler temperature from 200 to 350°C, subsequently treating the distillate (boiling range at a pressure of, for example, 0.2 mbar from 230 to 270°C) for from 10 to 60 minutes with from 0.7 to 10 times its weight of an organic solvent at a temperature from 70 to 180°C, if required separating the clear solution from undissolved components, cooling the removed clear solution to a temperature from 45 to 65°C and allowing it to crystallize at this temperature, which results in a suspension of dimethyl 4,4'-biphenyldicarboxylate crystals from which the crystals are isolated by conventional means, washed once or, if desired, one or more times with the same or a different solvent, and are dried.

2. The process as claimed in claim 1, wherein the organic solvent used is an alkanol having from 1 to 8 carbon atoms.

3. The process as claimed in at least one of claims 1 and 2, wherein the organic solvent used is methanol.

4. The process as claimed in at least one of claims 1 to 3, wherein distillation is carried out at a boiler temperature from 200 to 310°C.

5. The process as claimed in at least one of claims 1 to 4, wherein the distillate is treated with from 1 to 5 times its amount of the organic solvent.

6. The process as claimed in at least one of claims 1 to 5, wherein the treatment of the distillate with the organic solvent is carried out at from 70 to 150°C.

## Revendications

1. Procédé pour l'obtention de l'ester diméthylique de l'acide 4,4'-biphényl-dicarboxylique à partir des résidus et des courants latéraux de la préparation du DMT dans lequel on distille les résidus, respectivement la matière des courants latéraux d'abord sous une pression de tout au plus 7 mbars à une température du ballon de distillation de 200 à 350 °C, ensuite on traite le distillat (plage d'ébullition à une pression, par exemple de 0,2 mbar, dans l'intervalle de températures de 230 à 270 °C) pendant 10 à 60 minutes avec un excès 0,7 à 10 fois d'un solvant organique, à une température de 70 à 180 °C, si nécessaire on sépare la solution limpide des composants insolubles, on refroidit la solution limpide à une température de 45 à 65 °C et on laisse cristalliser à cette température, ce qui donne une suspension de cristaux d'ester diméthylique de l'acide 4,4'-biphényl-dicarboxylique, à partir de laquelle on isole les cristaux de façon usuelle, on lave à une ou plusieurs reprises, éventuellement avec le même ou un autre solvant, et on sèche.

2. Procédé selon la revendication 1, caractérisé en ce que l'on utilise comme solvant organique des alcanols avec 1 à 8 atomes de carbone.

3. Procédé selon au moins l'une des revendications 1 et 2, caractérisé en ce que l'on utilise comme solvant organique le méthanol.

4. Procédé selon au moins l'une des revendications 1 à 3, caractérisé en ce que l'on distille à une température du ballon de distillation de 200 à 310 °C.

5. Procédé selon au moins l'une des revendications 1 à 4, caractérisé en ce que l'on traite le distillat avec un excès 1 à 5 fois de solvant organique.

6. Procédé selon au moins l'une des revendications 1 à 5, caractérisé en ce que l'on traite le distillat avec le solvant organique à une température de 70 à 150 °C.
